# EUROPEAN PATENT APPLICATION

(11) **EP 4 678 223 A1**
(43) Date of publication of application: **14.01.2026**
(21) Application number: 24771110.4
(22) Date of filing: 07.03.2024
(51) Int. Cl.: A61N 1/32, A61N 1/36, A61N 1/04

(54) **SKIN CARE TOOL FOR EYE RIM SKIN CARE, AND EYE RIM SKIN CARE PROCEDURE METHOD USING SAME**

(30) Priority: 10.03.2023 KR 20230031931
(71) Applicant: APR CO., LTD., Seoul 05551 (KR)
(72) Inventor: LEE, Gyoun Jung, Seoul 08592 (KR); PARK, Seung Woo, Seoul 08592 (KR)
(74) Representative: SONN Patentanwälte GmbH & Co KG
(86) International application number: PCT/KR2024/002928
(87) International publication number: WO 2024/191110

(57) **Abstract**

A skin care tool for eye rim skin care, and an eye rim skin care procedure method using same are disclosed. The skin care tool for eye rim skin care, according to one aspect of the present invention, may comprise: a main body unit, a single electrode unit and a control unit. The eye rim skin care procedure method using the skin care tool for eye rim skin care, according to another aspect of the present invention, may comprise: an eye rim skin drying step; a shot mode procedure step; an effective skin component application step; and a booster mode procedure step.

## Description

### [TECHNICAL FIELD]

The present disclosure relates to a skin beauty apparatus, and more particularly, to a skin beauty apparatus for eye-area skin care, which can protect the eye-area skin of a subject and allow beauty treatment to be performed more safely on the eye-area skin even at home, and an eye-area skin care treatment method using the same.

### [BACKGROUND ART]

Generally, massage refers to rubbing, tapping, or kneading the skin with hands to improve skin elasticity or relieve muscle fatigue, and may be performed using bare hands or apparatuses.

Recently, various beauty apparatuses have become increasingly available, allowing users to perform such massage treatments by themselves at home.

These beauty apparatuses allow users to conveniently perform treatments such as massage at their desired time in their own homes, thereby lowering the barrier to beauty treatments and enhancing accessibility.

Meanwhile, the eye-area skin is the most delicate area of the facial skin.

However, various products, such as eye shadows, mascaras, eyeliners, and eyebrow pencils, have been developed to enhance beauty, and excessive use of these products can weaken the eye-area skin and inevitably place a significant burden on eye health.

Furthermore, the destruction of the ozone layer has increased the likelihood of exposure to ultraviolet rays; yellow dust and fine dust have increased exposure to heavy metals; and exposure to external pollutants, such as elevated carbon dioxide levels and harmful chemicals, has become more severe. As a result, the eye-area skin has become fragile, and its homeostasis has deteriorated. In addition, over time, wrinkles tend to form around the eyes more easily than in other facial areas.

As described above, the eye-area skin is a region that is repeatedly and continuously moved throughout life by the orbicularis oculi muscle. It is the thinnest skin on the human body and has a low density of sebaceous glands, causing skin elasticity to decrease rapidly, thereby making it the area where signs of aging appear most prominently. It is also a sensory-sensitive part that either covers or surrounds the eyeball, which is an important organ.

Accordingly, in managing the condition of facial skin, the eye-area skin is both one of the most important and one of the most difficult areas to care for.

Therefore, to manage the eye-area skin even at home, the skin beauty apparatus used needs to be prepared and implemented with functions that are more precise and delicate than those for performing beauty treatments on other facial areas.

As an apparatus for managing and massaging the eye-area skin, Korean Patent Laid-Open Publication No. 10-2017-0017411 discloses an eye massager. However, such a conventional apparatus is a simple massager utilizing vibration, making it difficult to provide fine care for the eye-area skin and unsuitable for wrinkle improvement or beauty treatments.

Furthermore, Korean Patent Laid-Open Publication No. 10-2023-0087324 discloses an eyeglass-type massager that applies low-frequency microcurrent electrical muscle stimulation (EMS) signals to the muscles of the eye-area skin, and Korean Patent Laid-Open Publication No. 10-2018-0020008 discloses a stimulation apparatus that promotes transdermal drug delivery to the skin using frequency pulses.

However, such conventional apparatuses not only have complicated structures but also include separate configurations for performing skin stimulation and drug administration functions, which makes them unsuitable and inconvenient for home use.

In addition, the eye-area skin requires delicate beauty treatment, but its structural characteristics make such treatment difficult to perform.

### [DISCLOSURE]

### [TECHNICAL PROBLEM]

The present disclosure aims to solve the above problems by providing a skin beauty apparatus for eye-area skin care, which can protect the eye-area skin of a subject and allow beauty treatment to be performed more safely on the eye-area skin even at home, and an eye-area skin care treatment method using the same.

Another object of the present disclosure is to provide a skin beauty apparatus for eye-area skin care, which not only induce collagen synthesis around the eye-area skin to improve wrinkles but also maximize the skin penetration effect of effective skin-care ingredients, and an eye-area skin care treatment method using the same.

Still another object of the present disclosure is to provide a skin beauty apparatus for eye-area skin care, which applies a single electrode having a ball-shaped electrode head to maximize safety for the eye-area skin and to maximize the beauty treatment effect on the eye-area skin through two different types of electrical stimulation, and an eye-area skin care treatment method using the same.

The problems of the present disclosure are not limited to those mentioned above, and other problems not mentioned will be clearly understood by those of ordinary skill in the art from the following description.

### [TECHNICAL SOLUTION]

According to an aspect of the present disclosure, there is provided a skin beauty apparatus for eye-area skin care.

The skin beauty apparatus for eye-area skin care may include a graspable main body; a single electrode having a ball-shaped electrode head at one side of the main body; and a controller provided in the main body.

In this case, the single electrode may perform a shot mode treatment by passing close to an eye-area skin of a subject at a position spaced apart by a preset distance from the eye-area skin and applying a high-voltage electrical stimulation to the eye-area skin according to a control signal of the controller, or perform a booster mode treatment by contacting the eye-area skin of the subject and applying a low-voltage electrical stimulation, which is lower than the high voltage of the shot mode treatment, to the eye-area skin according to a control signal of the controller.

In this case, the main body part may include a power terminal connected to an external power supply, or a battery.

In this case, the main body may include a switch electrically connected to the controller at one side thereof.

In this case, the switch may include a power switch for applying power, and a mode switch for selecting the shot mode treatment or the booster mode treatment.

If necessary, the main body may further include a lens disposed adjacent to the single electrode, and a communicator configured to transmit an image of the subject's eye-area skin captured through the lens to an external display via a wired or wireless network.

Meanwhile, the main body may further include an indicator configured to notify a usage state.

In addition, the indicator may indicate any one of a light emission signal, a vibration signal, or a sound signal as a notification signal.

According to another aspect of the present disclosure, an eye-area skin care treatment method using a skin beauty apparatus for eye-area skin care is provided.

The eye-area skin care treatment method using a skin beauty apparatus for eye-area skin care employs a graspable main body; a single electrode having a ball-shaped electrode head at one side of the main body; and a controller provided in the main body.

In this case, the eye-area skin care treatment method using a skin beauty apparatus for eye-area skin care may include an eye-area skin drying step of removing a moisture from a surface layer of an eye-area skin of a subject to form a dry state; a shot mode treatment step of spacing the single electrode apart from the subject's eye-area skin by a preset distance, passing close to the eye-area skin, and emitting a preset high voltage to the eye-area skin; an effective skin-care ingredient application step of applying an effective skin-care ingredient to the eye-area skin having undergone the shot mode treatment step; and a booster mode treatment step of contacting the single electrode with the subject's eye-area skin to which the effective skin-care ingredient has been applied and emitting a preset low voltage to the eye-area skin.

In this case, the high voltage in the shot mode treatment step is higher than the low voltage in the booster mode treatment step.

For example, the shot mode treatment step may include a shot mode treatment execution process of generating a preset high voltage according to a selection signal of the switch; a process of spacing the single electrode apart from the subject's eye-area skin by a preset distance, passing close to the eye-area skin, and generating a plurality of thermal sparks in the surface layer of the eye-area skin in the dry state after the moisture has been removed, by applying an electrical stimulation of the high voltage; a process of forming micro wounds in the surface layer of the eye-area skin by the thermal sparks; and a process of inducing collagen synthesis in the eye-area skin while the micro wounds are healed.

In this case, the shot mode treatment execution process may include generating the preset high voltage through a high-voltage micro thermal spark generation circuit provided in the controller.

For example, the booster mode treatment step may include a booster mode treatment execution process of generating a preset low voltage according to a selection signal of the switch; a process of contacting the single electrode with the eye-area skin to which the effective skin-care ingredient has been applied and forming an electric hole in the surface of the eye-area skin to allow molecular-level penetration by applying an electrical stimulation of the low-voltage; and a process of penetrating the applied effective skin-care ingredient through the electric hole while rubbing the surface layer of the eye-area skin with the single electrode.

Meanwhile, the shot mode treatment step, the effective skin-care ingredient application step, and the booster mode treatment step may further include a treatment confirmation step of transmitting an image of the eye-area skin captured through a lens disposed adjacent to the single electrode to an external display via a wired or wireless network for confirmation.

### [ADVANTAGEOUS EFFECTS]

According to the above configuration, the skin beauty apparatus for eye-area skin care and the eye-area skin care treatment method using the same according to the present disclosure can provide safe and suitable electrical stimulation even to the curved and narrow eye-area skin by forming an end of the single electrode as a ball-shaped electrode head, thereby further increasing safety and maximizing the beauty treatment effect.

In addition, since both a high-voltage shot mode treatment and a low-voltage booster mode treatment can be performed using one skin beauty apparatus, it has the effect of enabling easy beauty treatment of the eye-area skin even at home.

Further, the shot mode treatment can maximize the wrinkle improvement effect by generating minute micro thermal sparks at high voltage to momentarily stimulate the epidermal layer of the eye-area skin and induce collagen synthesis beneath the epidermal layer.

Furthermore, since the electrode head of the single electrode has a ball shape, it can apply electrical stimulation over a smooth and wide area compared to a conventional sharp tip, thereby preventing unnecessary skin damage to the eye-area skin.

In addition, the single electrode can deliver electrical stimulation deeper into the skin layers, enabling more precise beauty treatment tailored to the characteristics of the eye-area skin.

In addition, since the electrode head of the single electrode has a ball shape, it can conform to the curved and narrow skin shape around the eyes, enabling beauty treatment to be performed in any direction during the treatment and thereby optimizing the beauty treatment for the eye-area skin.

In addition, the apparatus can perform both a shot mode treatment, which instantaneously stimulates the eye-area skin to create and heal micro wounds, and a booster mode treatment, which reduces erythema, heat sensation, and pain on the skin applied with effective skin-care ingredients while aiding absorption of the effective ingredients, thereby effectively performing skin soothing and regeneration of the eye-area skin.

Advantageous effects of the present disclosure are not limited to the above-described effects and should be understood to include all effects that can be inferred from the configuration of the invention described in the detailed description or claims of the present disclosure.

### [DESCRIPTION OF DRAWINGS]

FIG. 1 is a schematic diagram showing a skin beauty apparatus for eye-area skin care according to an embodiment of the present disclosure.
FIG. 2 is a schematic diagram showing an internal component provided in the skin beauty apparatus for eye-area skin care according to an embodiment of the present disclosure.
FIG. 3 is a schematic diagram showing a shot mode treatment using the skin beauty apparatus for eye-area skin care according to an embodiment of the present disclosure.
FIG. 4 is a schematic diagram showing a booster mode treatment using the skin beauty apparatus for eye-area skin care according to an embodiment of the present disclosure.
FIG. 5 is a schematic diagram showing a skin beauty apparatus for eye-area skin care according to another embodiment of the present disclosure.
FIG. 6 is a schematic flowchart showing an eye-area skin care treatment method using a skin beauty apparatus for eye-area skin care according to another embodiment of the present disclosure.

### [BEST EMBODIMENTS]

The present disclosure, in its best mode, provides a skin beauty apparatus for eye-area skin care comprising: a graspable main body; a single electrode having a ball-shaped electrode head at one side of the main body; and a controller provided in the main body.

In this case, the single electrode may perform a shot mode treatment by passing close to an eye-area skin of a subject at a position spaced apart by a preset distance from the eye-area skin and applying a high-voltage electrical stimulation to the eye-area skin according to a control signal of the controller, or perform a booster mode treatment by contacting the eye-area skin of the subject and applying a low-voltage electrical stimulation, which is lower than the high-voltage of the shot mode treatment, to the eye-area skin according to a control signal of the controller.

Furthermore, the present disclosure, in its best mode, provides an eye-area skin care treatment method using a skin beauty apparatus for eye-area skin care, the method comprising: an eye-area skin drying step of removing a moisture from a surface layer of an eye-area skin of a subject to form a dry state; a shot mode treatment step of spacing the single electrode apart from the subject's eye-area skin by a preset distance, passing close to the eye-area skin, and emitting a preset high voltage to the eye-area skin; an effective skin-care ingredient application step of applying an effective skin-care ingredient to the eye-area skin having undergone the shot mode treatment step; and a booster mode treatment step of contacting the single electrode with the subject's eye-area skin to which the effective skin-care ingredient has been applied and emitting a preset low voltage to the eye-area skin.

### [DETAILED DESCRIPTION OF THE EMBODIMENTS]

Hereinafter, exemplary embodiments of the present disclosure will be described in detail so that those of ordinary skill in the art can readily implement the present disclosure with reference to the accompanying drawings. The present disclosure may be embodied in many different forms and is not limited to the embodiments set forth herein. In the drawings, parts unrelated to the description are omitted for clarity in describing the present disclosure, and throughout the specification, the same or similar reference numerals denote the same or similar elements.

The words and terms used in the present specification and claims should not be construed as being limited to their ordinary or dictionary meanings, but should be construed as having meanings and concepts consistent with the technical spirit of the present disclosure, in accordance with the principle that an inventor may define terms and concepts to best describe the present disclosure.

Accordingly, the embodiments described in the present specification and the configurations shown in the drawings correspond to preferred embodiments of the present disclosure, and do not represent all the technical idea of the present disclosure, so the configurations may have various examples of equivalent and modification that can replace them at the time of filing the present disclosure.

It should be understood that the terms "comprise" or "include" or "have" and the like, when used in the present specification, are intended to specify the presence of stated features, numbers, steps, operations, elements, components, or combinations thereof, but do not preclude the possibility of the presence or addition of one or more other features, numbers, steps, operations, elements, components, or combinations thereof.

The presence of an element in/on "front", "rear", "upper or above or top" or "lower or below or bottom" of another element includes not only being disposed in/on "front", "rear", "upper or above or top" or "lower or below or bottom" directly in contact with other elements, but also cases in which another element being disposed in the middle, unless otherwise specified. In addition, unless otherwise specified, the expression that an element is "connected" to another element should be construed to include not only a case of being directly connected to each other but also a case of being indirectly connected to each other.

In addition, in describing the present disclosure, detailed descriptions of related known functions or configurations will be omitted so as not to obscure the gist of the present disclosure.

Hereinafter, a skin beauty apparatus for eye-area skin care and an eye-area skin care treatment method using the same will be described with reference to the drawings.

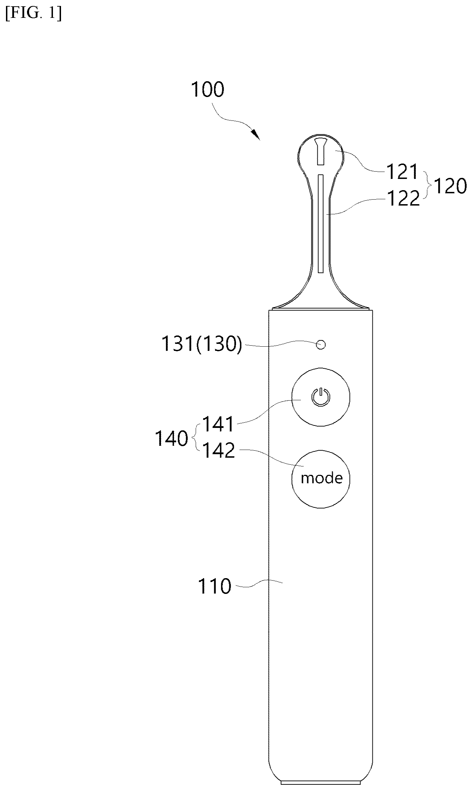

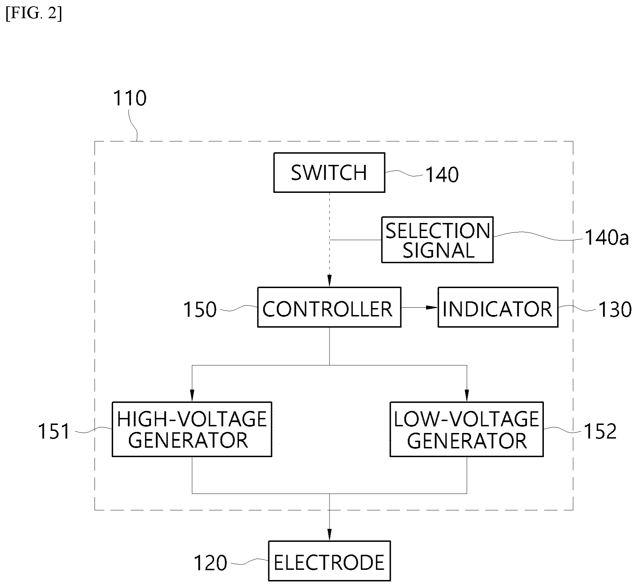

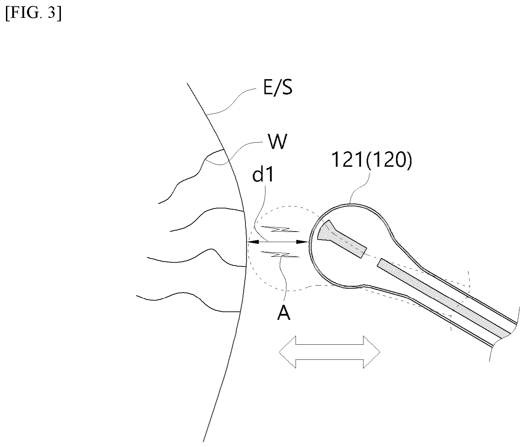

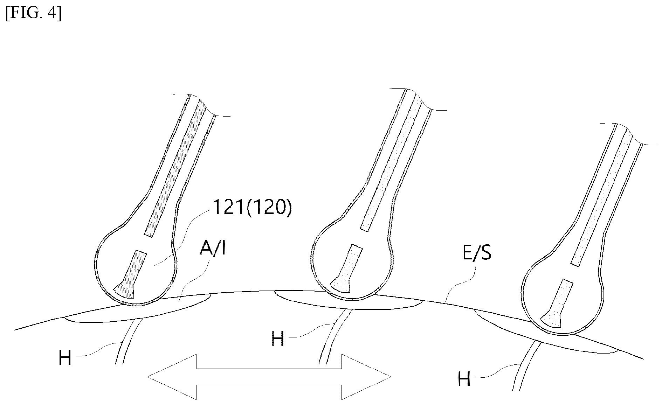

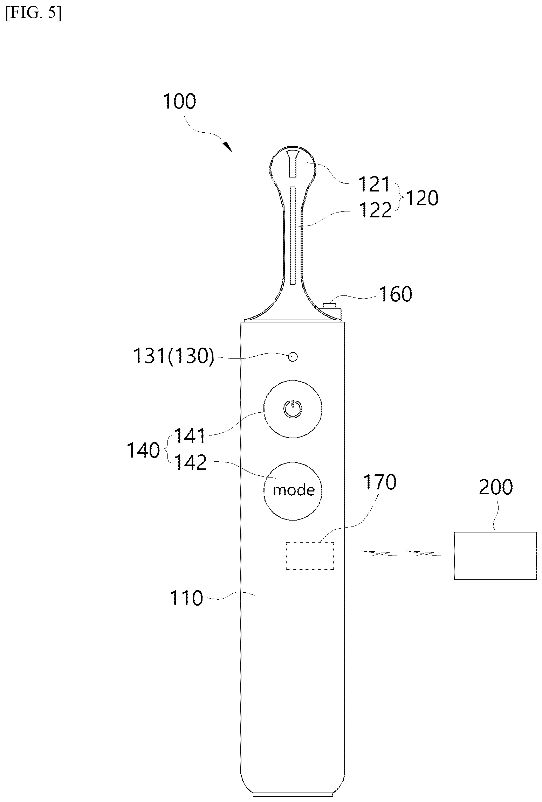

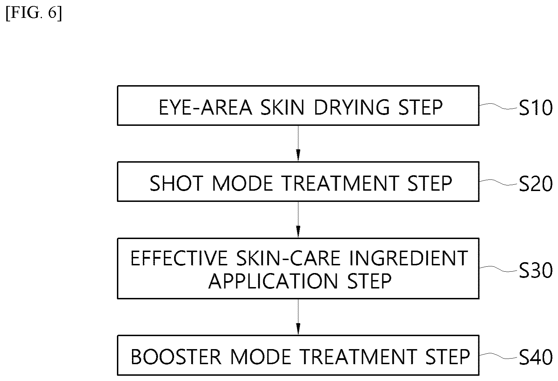

FIG. 1 is a schematic diagram showing a skin beauty apparatus for eye-area skin care according to an embodiment of the present disclosure, FIG. 2 is a schematic diagram showing an internal component provided in the skin beauty apparatus for eye-area skin care according to an embodiment of the present disclosure, and FIG. 3 is a schematic diagram showing a shot mode treatment using the skin beauty apparatus for eye-area skin care according to an embodiment of the present disclosure. In addition, FIG. 4 is a schematic diagram showing a booster mode treatment using the skin beauty apparatus for eye-area skin care according to an embodiment of the present disclosure, and FIG. 5 is a schematic diagram showing a skin beauty apparatus for eye-area skin care according to another embodiment of the present disclosure. FIG. 6 is a schematic flowchart showing an eye-area skin care treatment method using a skin beauty apparatus for eye-area skin care according to another embodiment of the present disclosure.

As shown, the skin beauty apparatus for eye-area skin care according to an embodiment of the present disclosure and the eye-area skin care treatment method using the same are configured to protect a subject's eye-area skin E/S (FIG. 3) while allowing two types of beauty treatments to be safely performed on the eye-area skin even at home.

To this end, referring to FIGS. 1 and 2, the skin beauty apparatus 100 for eye-area skin care according to an embodiment of the present disclosure mainly includes a main body 110, a single electrode 120, and a controller 150.

In addition, the main body 110 may further include a switch 140 configured to allow power supply or select between the shot mode treatment and the booster mode treatment, and an indicator 130 configured to check a usage state of the skin beauty apparatus 100, at one side thereof.

More specifically, the main body 110 has a shape graspable by a user. For example, the main body 110 may have a straight pen shape or a polygonal shape with a separately provided handle.

The main body 110 is not limited to a predetermined shape and may be variously shaped as long as the user can perform the shot mode treatment or booster mode treatment to be described later while gripping the main body 110 and applying it to a subject to be treated, which may be the user or another person.

However, since the shot mode treatment and booster mode treatment of the present disclosure are electrical stimulation treatments, the main body 110 is preferably illustrated to have a straight pen shape for safe and precise beauty treatments.

Meanwhile, the main body 110 includes a controller 150 therein and may be equipped with a power terminal (not shown) configured to be connected to an external power source as necessary for power supply or may include a battery (not shown). The battery may be rechargeable, and accordingly, the skin beauty apparatus 100 of the present disclosure may be in a wired or wireless form.

Further, the skin beauty apparatus 100 for eye-area skin care according to an embodiment of the present disclosure includes an electrode 120 at one side of the main body 110 to apply a high-voltage or low-voltage electrical stimulation to the eye-area skin E/S.

The electrode 120 may be referred to as a monopolar single electrode 120, and the single electrode 120 includes an electrode head 121 at its end.

Meanwhile, as described above, the present disclosure performs a beauty treatment on the eye-area skin E/S of the subject. Accordingly, the electrode head 121 may preferably have a ball shape.

In skin beauty treatments using electrical stimulation, electrode heads typically have pointed tips, and such pointed tips may cause unnecessary skin damage to the eye-area skin E/S due to carelessness. In addition, such pointed tip shapes cannot perform massage functions such as rubbing the eye-area skin E/S.

To solve these problems and to apply electrical stimulation to the skin with a smoother and wider surface area compared to pointed tips, the single electrode 120 of the skin beauty apparatus 100 according to the present disclosure is characterized by including a ball-shaped electrode head 121.

Additionally, the single electrode 120 can apply an electrical stimulation deeper to the eye-area skin E/S compared to bipolar electrodes, thereby applying instantaneous stimulation to the surface layer (epidermis) of the eye-area skin E/S while preventing unnecessary skin damage and applying the electrical stimulation to a desired location.

Meanwhile, if necessary, the single electrode 120 may have a structure including a stick 122 having a preset length at one end of the main body 110 and the above-described ball-shaped electrode head 121 at an end of the stick 122. The overall length of the single electrode 120 may be set by the length of the stick 122.

Meanwhile, the controller 150 is provided in the main body 110 and includes a microcontroller unit (MCU) and a high-voltage micro thermal spark generation circuit. Accordingly, the controller 150 may generate a control signal to generate a high voltage or a low voltage through the high-voltage micro thermal spark generation circuit.

In other words, the controller 150 includes a high-voltage generator 151 and a low-voltage generator 152 (see FIG. 2).

According to a control signal of the controller 150, the high-voltage micro thermal spark generation circuit functions as the high-voltage generator 151 to generate a high voltage and perform a shot mode treatment using the single electrode 120.

In addition, according to a control signal from the controller 150, the high-voltage micro thermal spark generation circuit may generate a low voltage via the low-voltage generator 152 and perform a booster mode treatment using the single electrode 120.

In other words, the single electrode 120 performs a shot mode treatment using a high voltage, passing close to an eye-area skin E/S of a subject at a position spaced apart by a preset distance d1 from the eye-area skin E/S (see FIG. 3) according to a control signal from the controller 150.

In addition, the single electrode 120 performs a booster mode treatment at a position contacting the subject's eye-area skin E/S, by applying a low-voltage electrical stimulation as compared with a high voltage, according to a control signal of the controller 150 (see FIG. 4).

Such shot mode treatment and booster mode treatment will be described in more detail below.

Meanwhile, as described above, the skin beauty apparatus 100 for eye-area skin care according to an embodiment of the present disclosure includes a switch 140 electrically connected to the controller 150 at one side of the main body 110.

The switch 140 may have a structure including a power switch 141 for supplying power through an external power source or a battery, and a mode switch 142 for selecting the shot mode treatment or the booster mode treatment.

The mode switch 142 may allow a user of the skin beauty apparatus 100 to select the treatment mode by selectively pressing it or may be set to automatically change the treatment mode after a preset time elapses. In addition, the power switch 141 may be set to automatically cut off power supply after a set time elapses so that the treatment mode is not performed.

This is because the skin beauty apparatus 100 of the present disclosure performs a skin care treatment through electrical stimulation, thereby preventing skin damage caused by excessive treatment time and allowing safer skin care treatment even at home.

To this end, the controller 150 may further include a timer (not shown), and one side of the main body 110 may further include a separate switch for adjusting the time set in the timer, or the above-described power switch 141 or mode switch 142 may further perform such a function.

Meanwhile, the main body 110 may further include an indicator 130 on one side to indicate externally that the skin beauty apparatus 100 of the present disclosure is in use, that is, in a state ready for or undergoing the treatment.

In this case, the indicator 130 may generate a notification signal according to a control signal from the controller 150.

If necessary, such a notification signal may be any one of a light-emitting signal indicating the usage state by blinking light, a vibration signal indicating the usage state by vibrating a vibration element, or a sound signal indicating the usage state by outputting a pre-stored voice or sound via a speaker.

Further, if necessary, the usage state may be indicated by a single notification signal or by multiple notification signals.

Meanwhile, referring again to FIG. 5, the skin beauty apparatus 100 for eye-area skin care according to another embodiment of the present disclosure enables a user or subject to more clearly confirm the skin care treatment state of the eye-area skin E/S through an external display 200.

To this end, the main body 110 of the skin beauty apparatus 100 for eye-area skin care according to another embodiment of the present disclosure may further include a lens 160 disposed adjacent to the single electrode 120 to capture the eye-area skin E/S undergoing the skin care treatment.

Further, the main body 110 may further include a communicator 170 configured to transmit, via a wired or wireless network, an image of the subject's eye-area skin E/S captured through the above-described lens 160 to the external display 200.

It is to be understood that the image transmission of the communicator 170 may be controlled by the controller 150. In addition, whether to transmit the image may also be selected through mode operation of the switch 140.

Meanwhile, referring again to FIGS. 1 to 5 and then to FIG. 6, an eye-area skin care treatment method using the skin beauty apparatus for eye-area skin care according to an embodiment of the present disclosure will be described in detail below.

As described above, the skin beauty apparatus 100 for eye-area skin care according to an embodiment of the present disclosure may stably and continuously perform two types of skin care treatments on the eye-area skin E/S using one single electrode 120.

First, before describing the eye-area skin care treatment method, the shot mode treatment of the skin beauty apparatus 100 for eye-area skin care according to an embodiment of the present disclosure will be described as follows.

The skin beauty apparatus 100 for eye-area skin care of the present disclosure performs a shot mode treatment using a high voltage, repeatedly passing the single electrode 120 close to the subject's eye-area skin E/S from a position spaced apart by a preset distance d1 (see FIG. 3) according to a control signal from the controller 150.

Such a shot mode treatment is one type of treatment using electrical stimulation, in which the single electrode 120 is placed at a position spaced apart from the eye-area skin E/S and repeatedly passes close to the eye-area skin E/S, generating a fine micro thermal spark A with an instantaneous high voltage to momentarily stimulate the surface layer (epidermis) of the eye-area skin E/S.

Through the shot mode treatment, collagen synthesis is induced beneath the epidermal layer of the eye-area skin E/S, thereby providing an effect of improving wrinkles.

When the single electrode 120 is placed at a position spaced apart from the eye-area skin E/S and a high-voltage is momentarily applied while repeatedly rubbing the eye-area skin E/S, a fine micro thermal spark A is momentarily generated between the eye-area skin E/S and an electrode head 121 of the single electrode 120.

Such a thermal spark A generates heat and creates fine wounds in the eye-area skin E/S, where healing regeneration to recover the fine wounds occurs. In this process, collagen is densely located in the new tissue compared to the old skin, which can be regarded as inducing collagen synthesis.

Through such induction of collagen synthesis, an effect of improving wrinkles W around the eye-area skin E/S (see FIG. 3) may be obtained.

Meanwhile, since the electrode head 121 of the single electrode 120 is designed in a ball-type rounded shape, it may conform to the curved and narrow shape of the eye-area skin and apply electrical stimulation uniformly in any direction during the shot mode treatment.

Next, before describing the eye-area skin care treatment method, the booster mode treatment of the skin beauty apparatus 100 for eye-area skin care according to the embodiment of the present disclosure will be described as follows.

The skin beauty apparatus 100 for eye-area skin care of the present disclosure performs a booster mode treatment using a low voltage, compared to the high voltage of the shot mode treatment, at a position in contact with the eye-area skin E/S of the subject (see FIG. 4).

The booster mode treatment is one type of treatment using electrical stimulation, namely electroporation, which promotes absorption by delivering the active components of cosmetics or drugs containing effective skin-care ingredient into the eye-area skin E/S.

In other words, when the single electrode 120 is placed in contact with the eye-area skin E/S and a voltage lower than that of the shot mode is momentarily applied, an electric hole H (see FIG. 4) is formed in the surface layer (epidermis) of the eye-area skin E/S, enabling molecular-level penetration and thereby allowing effective skin-care ingredients to permeate and promoting absorption.

Such a booster mode treatment helps the effective skin-care ingredients to be absorbed into the skin by applying the effective skin-care ingredients, such as cosmetics, to the eye-area skin E/S and then rubbing the area as if massaging.

Meanwhile, such a booster mode treatment has the advantage of easily delivering effective skin-care ingredients, such as hydrophilic components or polymer components that cannot penetrate the skin from the epidermis, deep into the skin, because it temporarily forms an electric hole H (see FIG. 4) leading into the skin without using a needle. Accordingly, the absorption rate of effective skin-care ingredients is very high. That is, components with large particles, such as hyaluronic acid or collagen, can easily penetrate the skin. Furthermore, since there is no pH change, it can act more mildly on the skin.

In addition, since the electrode head 121 of the single electrode 120 is designed in a ball-type rounded shape, it may conform to the curved and narrow shape of the eye-area skin, allowing electrical stimulation to be applied uniformly in any direction during the booster mode treatment, and is easy to massage, making it highly suitable for eye-area skin care.

As described above, the skin beauty apparatus 100 for eye-area skin care according to an embodiment of the present disclosure may perform both a shot mode treatment, which instantaneously stimulates the eye-area skin E/S to create and heal micro wounds, and a booster mode treatment, which reduces erythema, heat sensation, and pain on the skin applied with effective skin-care ingredients while aiding absorption of the effective ingredients, thereby effectively performing skin soothing and regeneration of the eye-area skin.

As described above, the eye-area skin care treatment method performing the shot mode treatment and the booster mode treatment will be described in more detail as follows.

The eye-area skin care treatment method using the skin beauty apparatus for eye-area skin care according to an embodiment of the present disclosure comprises the following steps.

Referring to FIG. 6, the eye-area skin care treatment method using the skin beauty apparatus for eye-area skin care generally includes an eye-area skin drying step (S10), a shot mode treatment step (S20), an effective skin-care ingredient application step (S30), and a booster mode treatment step (S40).

First, the eye-area skin drying step (S10) is a step of removing moisture from the surface layer of the eye-area skin E/S of the subject using a moisture-absorbing paper or a dry towel to form a dry state.

This eye-area skin drying step (S10) is for maximizing the effect of the shot mode treatment to be described later, preventing thermal sparks generated during the shot mode treatment from creating unnecessary wounds on areas other than the eye-area skin E/S, thereby enabling the skin care treatment to be concentrated on the eye-area skin.

Meanwhile, if necessary, the eye-area skin drying step (S10) may be performed using a separate drying device such as a hair dryer.

Subsequently, the shot mode treatment step (S20) is performed.

The shot mode treatment step (S20) is a step of spacing the single electrode 120 apart from the eye-area skin E/S of the subject by a preset distance d1, passing close to the eye-area skin E/S again, and emitting a preset high voltage to the eye-area skin E/S according to a control signal from the controller 150.

The preset distance d1 may of course be set according to the condition of the eye-area skin and the voltage.

Specifically, this shot mode treatment step (S20) includes a power application process using the power switch 141 of the switch 140.

Further, according to a selection signal 140a of the mode switch 142 of the switch 140, the apparatus has a shot mode treatment execution process in which the controller 150 generates a high voltage. This shot mode treatment execution process generates the preset high voltage through a high-voltage micro thermal spark generation circuit provided in the controller 150.

Then, the single electrode 120 is spaced apart from the eye-area skin E/S by the preset distance d1, passing close to the eye-area skin E/S, and generates a plurality of thermal sparks in the surface layer of the eye-area skin E/S in a dry state after moisture has been removed, by applying the high-voltage electrical stimulation.

In this case, micro wounds are formed in the surface layer of the eye-area skin E/S by the thermal sparks.

Further, as the micro wounds are healed, a process of inducing collagen synthesis may be continuously performed in the eye-area skin E/S.

Subsequently, an effective skin-care ingredient application step (S30) is performed on the eye-area skin E/S that has undergone the shot mode treatment step (S20).

The effective skin-care ingredient application step (S30) is a step of applying an ampoule containing ingredients effective for skin beauty to the eye-area skin E/S, and may be performed by dropping the effective skin-care ingredient onto the eye-area skin E/S. In addition, if necessary, a separate vibration mechanism may be used to apply the ingredient over the entire facial skin, including the eye-area.

Further, although not illustrated, the skin beauty apparatus 100 for eye-area skin care according to an embodiment of the present disclosure may include a separate vibration motor to apply effective skin-care ingredients to the eye-area skin E/S through vibration.

The effective skin-care ingredient is not limited to any particular type and may include an ingredient containing hyaluronic acid or collagen.

Subsequently, the booster mode treatment step (S40) for the eye-area skin E/S is performed.

The booster mode treatment step (S40) is a step of contacting the ball-shaped electrode head 121 of the single electrode 120 with the subject's eye-area skin E/S to which the effective skin-care ingredient has been applied and emitting a low voltage to the eye-area skin E/S according to a control signal of the controller 150.

In this case, as shown in FIG. 4, the single electrode 120 is moved as if massaging to further maximize the treatment effect.

Specifically, this booster mode treatment step (S40) includes a booster mode treatment execution process of generating a low voltage in the controller 150 according to the selection signal 140a of the mode switch 142 of the switch 140. This booster mode treatment execution process generates the preset low voltage through the high-voltage micro thermal spark generation circuit provided in the controller 150.

Meanwhile, the terms high voltage and low voltage, that is, high voltage, low voltage, high frequency, and low frequency, are relative concepts used to help understand the electrical stimulation applied in different electrical treatments within the same apparatus.

In other words, the high voltage in the above-described shot mode treatment step (S20) means a voltage higher than the low voltage in the booster mode treatment step (S40) and is not limited to any specific voltage and may be set to any arbitrary voltage as necessary. Likewise, the low voltage in the booster mode treatment step (S40) means a voltage lower than the high voltage in the shot mode treatment step (S20) and is not limited to any specific voltage and may be set to any arbitrary voltage as necessary.

Subsequently, after the booster mode treatment execution process, the booster mode treatment step (S40) includes a process of contacting the electrode head 121 of the single electrode 120 with the eye-area skin E/S to which the effective skin-care ingredient has been applied, and forming an electric hole H (see FIG. 4) on the surface of the eye-area skin E/S, the electric hole being capable of molecular-level penetration through electrical stimulation using the preset low voltage.

Then, a process of penetrating the effective skin-care ingredient through the electric hole H while rubbing the surface layer of the eye-area skin E/S with the single electrode 120 is performed. This facilitates absorption of the effective skin-care ingredient into the eye-area skin E/S.

Meanwhile, according to the step in which the eye-area skin care treatment method using the skin beauty apparatus 100 for eye-area skin care is performed, the skin beauty apparatus 100 indicates externally, through the indicator 130, that it is in use, that is, in a state ready for or undergoing the treatment.

The notification of the indicator 130 may be referred to as a notification step, which is performed upon operation of the switch 140 and, of course, may be performed simultaneously with the eye-area skin drying step (S10), the shot mode treatment step (S20), the effective skin-care ingredient application step (S30), and booster mode treatment step (S40).

In this case, the notification of the indicator 130 generates a notification signal according to a control signal of the controller 150, and if necessary, such a notification signal may be any one of a light-emitting signal indicating the usage state by blinking a lamp 131 (FIG. 1), a vibration signal indicating the usage state by vibrating a vibration element, or a sound signal indicating the usage state by outputting a pre-stored voice or sound via a speaker.

Meanwhile, the eye-area skin care treatment method using the skin beauty apparatus 100 for eye-area skin care according to an embodiment of the present disclosure may further include a treatment confirmation step of transmitting an image of the eye-area skin captured through a lens 160 (FIG. 5) disposed adjacent to the single electrode 120 to an external display 200 (FIG. 5) via a wired or wireless network for confirmation.

This treatment confirmation step may be included in and performed together with the shot mode treatment step (S20), the effective skin-care ingredient application step (S30), and the booster mode treatment step (S40).

Through this treatment confirmation step, the user can perform the eye-area skin care treatment more safely.

As described above, the skin beauty apparatus for eye-area skin care and the eye-area skin care treatment method using the same according to the present disclosure include an end portion of the single electrode 120 formed as a ball-shaped electrode head 121, which can provide safe and suitable electrical stimulation even to the curved and narrow skin around the eye area, thereby enhancing stability and maximizing the skin care treatment effect.

In addition, two types of treatments, a high-voltage shot mode treatment and a low-voltage booster mode treatment, can be performed using one skin beauty apparatus 100, enabling easy performance of skin care treatment for the eye-area skin even at home.

Further, the shot mode treatment can maximize the wrinkle improvement effect by generating minute micro thermal sparks at high voltage to momentarily stimulate the epidermal layer and induce collagen synthesis beneath the epidermal layer.

Furthermore, since the electrode head 121 of the single electrode 120 has a ball shape, it can apply electrical stimulation over a smooth and wide area compared to a conventional sharp tip, thereby preventing unnecessary skin damage to the eye-area skin.

In addition, the single electrode 120 can deliver electrical stimulation deeper into the skin layers, enabling more precise beauty treatment tailored to the characteristics of the eye-area skin.

In addition, since the electrode head 121 of the single electrode 120 has a ball shape, it can conform to the curved and narrow skin shape around the eyes, enabling beauty treatment to be performed in any direction during the treatment and thereby optimizing the beauty treatment for the eye-area skin.

In addition, the shot mode treatment, which provides momentary stimulation to the eye-area skin E/S to create and heal micro wounds, and the booster mode treatment, which reduces erythema, heat sensation, and pain on the skin coated with effective skin-care ingredients and facilitates their absorption, can be performed together, thereby effectively achieving skin soothing and regeneration for the eye-area skin.

Although exemplary embodiments of the present disclosure have been described, the spirit of the present disclosure is not limited to the embodiments set forth herein. Those of ordinary skill in the art who understand the spirit of the present disclosure may easily propose other embodiments through supplement, change, removal, addition, etc. of elements within the same scope of spirit, but the embodiments will be also within the scope of the present disclosure.

## Claims

1. A skin beauty apparatus for eye-area skin care comprising:
a graspable main body;
a single electrode having a ball-shaped electrode head at one side of the main body; and
a controller provided in the main body,
wherein the single electrode is configured to:
perform a shot mode treatment by passing close to an eye-area skin of a subject at a position spaced apart by a preset distance from the eye-area skin and applying a high-voltage electrical stimulation to the eye-area skin according to a control signal of the controller, or
perform a booster mode treatment by contacting the eye-area skin of the subject and applying a low-voltage electrical stimulation, which is lower than the high-voltage of the shot mode treatment, to the eye-area skin according to a control signal of the controller.

2. The skin beauty apparatus of claim 1, wherein the main body includes a power terminal connected to an external power supply, or a battery.

3. The skin beauty apparatus of claim 1, wherein the main body includes a switch electrically connected to the controller at one side thereof.

4. The skin beauty apparatus of claim 3, wherein the switch includes:
a power switch for applying power, and
a mode switch for selecting the shot mode treatment or the booster mode treatment.

5. The skin beauty apparatus of claim 1, wherein the main body further includes:
a lens disposed adjacent to the single electrode, and
a communicator configured to transmit an image of the subject's eye-area skin captured through the lens to an external display via a wired or wireless network.

6. The skin beauty apparatus of claim 1, wherein the main body further includes:
an indicator configured to notify a usage state.

7. The skin beauty apparatus of claim 6, wherein the indicator indicates:
any one of a light emission signal, a vibration signal, or a sound signal as a notification signal.

8. An eye-area skin care treatment method using a skin beauty apparatus for eye-area skin care having a graspable main body; a single electrode having a ball-shaped electrode head at one side of the main body; and a controller provided in the main body, the method comprising:
an eye-area skin drying step of removing a moisture from a surface layer of an eye-area skin of a subject to form a dry state;
a shot mode treatment step of spacing the single electrode apart from the subject's eye-area skin by a preset distance, passing close to the eye-area skin, and emitting a preset high voltage to the eye-area skin;
an effective skin-care ingredient application step of applying an effective skin-care ingredient to the eye-area skin having undergone the shot mode treatment step; and
a booster mode treatment step of contacting the single electrode with the subject's eye-area skin to which the effective skin-care ingredient has been applied and emitting a preset low voltage to the eye-area skin.

9. The eye-area skin care treatment method of claim 8, wherein the high voltage in the shot mode treatment step is higher than the low voltage in the booster mode treatment step.

10. The eye-area skin care treatment method of claim 8, wherein the shot mode treatment step includes:
a shot mode treatment execution process of generating a preset high voltage according to a selection signal of the switch;
a process of spacing the single electrode apart from the subject's eye-area skin by a preset distance, passing close to the eye-area skin, and generating a plurality of thermal sparks in the surface layer of the eye-area skin in the dry state after the moisture has been removed, by applying an electrical stimulation of the high voltage;
a process of forming micro wounds in the surface layer of the eye-area skin by the thermal sparks; and
a process of inducing collagen synthesis in the eye-area skin while the micro wounds are healed.

11. The eye-area skin care treatment method of claim 10, wherein the shot mode treatment execution process includes:
generating the preset high voltage through a high-voltage micro thermal spark generation circuit provided in the controller.

12. The eye-area skin care treatment method of claim 8, wherein the booster mode treatment step includes:
a booster mode treatment execution process of generating a preset low voltage according to a selection signal of the switch;
a process of contacting the single electrode with the eye-area skin to which the effective skin-care ingredient has been applied and forming an electric hole in the surface of the eye-area skin to allow molecular-level penetration by applying an electrical stimulation of the low-voltage; and
a process of penetrating the applied effective skin-care ingredient through the electric hole while rubbing the surface layer of the eye-area skin with the single electrode.

13. The eye-area skin care treatment method of claim 8, wherein the shot mode treatment step, the effective skin-care ingredient application step, and the booster mode treatment step further include:
a treatment confirmation step of transmitting an image of the eye-area skin captured through a lens disposed adjacent to the single electrode to an external display via a wired or wireless network for confirmation.
